# EUROPEAN PATENT APPLICATION

(11) **EP 4 309 590 A1**
(43) Date of publication of application: **24.01.2024**
(21) Application number: 23186479.4
(22) Date of filing: 19.07.2023
(51) Int. Cl.: A61B 10/00, A61B 10/02, A61B 5/15

(54) **MICROSCOPE-READY SAMPLE COLLECTING DEVICE**

(30) Priority: 19.07.2022 GB 202210578
(71) Applicant: Apacor Limited, Wokingham, Berkshire RG41 2QL (GB)
(72) Inventor: Mansouri, Yasmine, Wokingham, RG41 2QL (GB); Miller, Andrew, Wokingham, RG41 2QL (GB); Bellm, Anthony, Wokingham, RG41 2QL (GB); Bromley, Alan, Wokingham Berkshire, RG41 2QL (GB)
(74) Representative: Coulson, Elizabeth Eve

(57) **Abstract**

A microscope-ready sample collecting device (10, 110, 210, 310) comprising a sample housing portion (80, 180, 280, 380); a sample collecting portion (90, 190, 290, 390) comprising a (16a/16b/36, 116a/116b/136, 216a/216b/236, 316a/316b/336); and a hinge element (24, 124, 224, 324) between the sample housing portion (80, 180, 280, 380) and sample collecting portion (90, 190, 290, 390) for foldably coupling the sample collecting portion to the sample housing portion.

## Description

### FIELD OF THE INVENTION

The present invention relates to a microscope-ready sample collecting device. The invention finds advantageous application in the collection and analysis of medical samples such as, parasites, ova, larvae, microorganisms, other pathogens, cells, tissue, blood and the like.

### BACKGROUND OF THE INVENTION

Pinworm (Enterobuis vermicularis) infections commonly occur in all parts of the world. It is the most common worm infection in the developed world (approx. 209 million people infected). School aged children are the most commonly infected. In the United States about 20% of people at one point in time develop pinworm and an estimated 30% of children worldwide. Infection rates among high-risk groups may be as high as 50%. It is not considered a serious disease and causes minor itching; however, treatment is recommended. For the appropriate treatment to be prescribed it is important to firstly diagnose the infection. For proper diagnosis it is necessary to take a sample of the anal area of the patient, in the morning, after the pinworm has secreted eggs. Typically, a swab with an adhesive tape affixed thereto is used. The sample is then retained in a screw-top tube for transportation to a laboratory for testing. At the laboratory, the test tube is unscrewed, the sample is removed from the tube; and the sample is then transferred to a microscope slide. These steps may be conducted by a lab-technician. Then, the microscope slide can be taken for microscope analysis where inspection of the sample is carried out, sometimes by a different analyst and the presence of pinworm, pinworm ova or other parasites can be determined.

The applicant has identified that it would be desirable if the time, labour, and resources involved in the lab-based processing could be made more efficient and safer with less risk of sample-contamination or infection of third parties. It would also be desirable if costs involved with transporting the sample from the patient to the lab could be reduced and if the safety of the sample-transportation could be improved. The applicant has identified the need for a more streamlined and efficient method for sampling a patient and processing the sample by microscopy.

Accordingly, the present invention seeks to provide an improvement in the field of sample taking and preparation of sample for microscope analysis that has particular application for diagnosing a pinworm infection. The invention may be utilised in applications other than for pinworm infection, for example it is foreseen that the invention may have application in a wide variety of diagnostics and may provide improvements to healthcare for humans and other animals. Furthermore, it is foreseen that the invention may have application beyond medical or healthcare requirements and may have beneficial application in the collection, protection, transportation and microscope analysis of a variety of samples in fields such as forensic science, archaeology, geology, metallurgy, chemistry, electronics, and others.

### SUMMARY OF THE INVENTION

Aspects of the invention provide a microscope-ready sample collecting device as claimed in the appended claims.

According to one aspect of the invention for which protection is sought, there is provided, a microscope-ready sample collecting device comprising: a sample housing portion; a sample collecting portion comprising a sample collecting head; and a hinge element between the sample housing portion and sample collecting portion for foldably coupling the sample collecting portion to the sample housing portion, the sample housing portion comprising:
i) a generally planar main body and first and second opposing side walls;
ii) a sample-viewing window defined on or in a first surface of the generally planar main body; and a
iii) a sample-housing defined by a wall upstanding from a second surface of the generally planar main body;

wherein the hinge element allows the sample collecting head to be folded relative to the sample housing portion such that the sample collecting head can be received within the sample housing and such that a sample collected using the sample collecting head is thereby presented relative to the sample-viewing window such that almost immediately after collection, the sample is disposed in readiness for microscope analysis;
wherein said wall of the sample housing portion is shaped and configured to enclose the sample collecting head; and
wherein said sample collecting head and said sample-housing are configured to co-operably mechanically fit together for protecting a collected sample and for mitigating against contamination and damage of the collected sample

Optionally, said sample collecting head and said sample-housing are configured to co-operably mechanically fit together by a connection means associated with the sample housing and/or the sample collecting head; the connection means associated with the sample housing and/or the sample collecting head being operable once the device is folded and enables the sample collecting head to be connected to the upstanding ridge of the sample housing for securing the sample collecting head in the folded, micorscope-ready position.

Optionally, the sample collecting head is stadium-shaped or paddle-shaped.

Optionally, the sample collecting portion comprises a neck.

Optionally, the neck has a narrower width than a maximum width of the sample collecting head.
Optionally, the upstanding wall defining the sample-viewing window is shaped and sized correspondingly to the shape and size of the paddle-shaped sample collecting head.

Optionally, the connection means takes the form of co-operating elements that snap-fit between the sample collecting head and the upstanding wall of the sample-housing portion.

Optionally, the hinge element is provided by a living hinge moulded between the neck and the planar body.

Optionally, on an outer surface, the neck comprises one or more ribs, ridges or other grip-enhancing mouldings.

Optionally, the sample collecting head comprises an adhesive coating on at least one side.

Optionally, the first and second opposing side walls of the generally planar main body each have a height of between about 5mm and about 20mm.

Optionally, on outer surfaces the first and second opposing side walls of the generally planar main body one or more ribs, ridges or other grip-enhancing mouldings are provided.

Optionally, the device is formed completely or substantially from plastic.

Optionally, upon the sample collecting portion being folded into a secure location within the folded sample housing portion, and the sample collecting head being properly disposed for inspection through the sample viewing window, the sample collecting head is secured to the sample housing for maintaining the collected sample in the correct location.

Optionally, the sample housing has a depth defined by the height of the upstanding wall. The upstanding wall may have a depth of between about 5mm and about 20mm.

Optionally, upon using the connection means to locate the folded sample collecting head in the sample housing, the connection means cannot readily, by hand, be disconnected, thereby providing a tamper-evident feature to protect the integrity of the collected sample.

Alternatively, a release tab is provided, such that upon using the connection means to locate the folded sample collecting head in the sample housing, the release tab is operable to disengage the connection means.
Optionally, the sample collector comprises a sharp portion.

Within the scope of this application, it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

Embodiments of the invention will now be described, by way of example only, with reference to the accompanying drawings, in which:
FIGURE 1 is a view of a first side of a microscope-ready sample collecting device, in an open unused state, according to a first embodiment of the invention;
FIGURE 2 shows a view of a second side of the microscope-ready sample collecting device of Figure 1 as well as a cross-sectional view through the line A-A as indicated on the plan-view of the second side of the device shown on the left-hand side of Figure 2;
FIGURE 3 shows perspective views of the first and second sides of the microscope-ready sample collecting device of Figures 1 and 2, both in an open condition, ready for sample-collection;
FIGURE 4 shows perspective views of the first and second sides of the microscope-ready sample collecting device of Figure 3, both in a closed condition, ready for transportation to a lab and analysis by microscopy. The collected sample is enclosed within the sample collecting device;
FIGURE 5 shows perspective views of the first and second sides of a microscope-ready sample collecting device according to a second embodiment of the invention, where, in both perspective views, the device is shown in an open condition;
FIGURE 6 shows: a view of a first side of the microscope-ready sample collecting device of the second embodiment of the invention, a side-view of that device; a plan-view of a second side of that device, and a cross-sectional view through the line A-A as indicated on the plan-view of the second side of the device shown;
FIGURE 7 shows a view of a first side and a perspective view of a microscope-ready sample collecting device according to a third embodiment of the invention;
FIGURE 8 shows perspective views of first and second sides of a microscope-ready sample collecting device according to a fourth embodiment of the invention, both in an open condition, ready for sample-collection; and FIGURE 8 shows perspective views of the first and second sides of the microscope-ready sample collecting device in a closed condition, ready for transportation and/or sample-analysis by microscopy. The collected sample is enclosed within the sample collecting device.

### DETAILED DESCRIPTION OF EMBODIMENTS

Detailed descriptions of specific embodiments of the microscope-ready sample collecting devices of the present invention are disclosed herein. It will be understood that the disclosed embodiments are merely examples of the way in which certain aspects of the invention can be implemented and do not represent an exhaustive list of all of the ways the invention may be embodied. Indeed, it will be understood that the microscope-ready sample collecting devices described herein may be embodied in various and alternative forms. The figures are not necessarily to scale and some features may be exaggerated or minimised to show details of particular components. Well-known components, materials or methods are not necessarily described in great detail in order to avoid obscuring the present disclosure. Any specific structural and functional details disclosed herein are not to be interpreted as limiting, but merely as a basis for the claims and as a representative basis for teaching one skilled in the art to variously employ the invention.

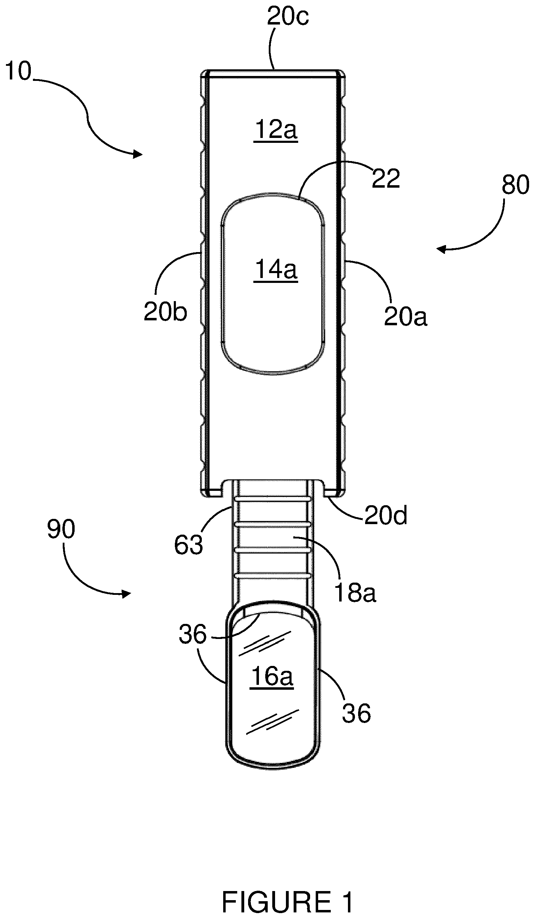

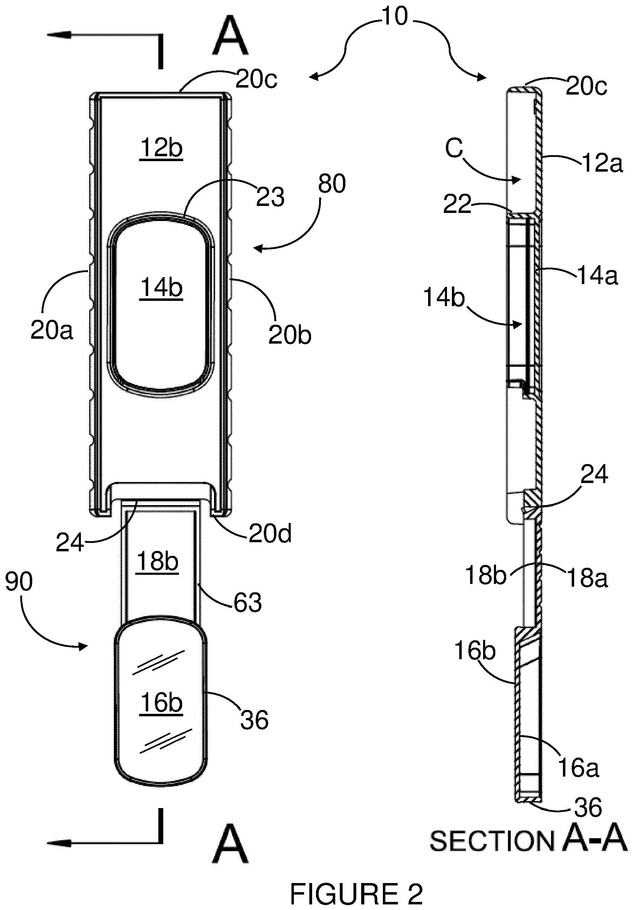

Referring to Figure 1, there is shown a top-view of a first side of a microscope-ready sample collecting device 10 according to a first embodiment of the invention. The microscope-ready sample collecting device 10 comprises a sample collecting portion 90 and a sample housing portion 80, having a hinge element 24 therebetween for foldably coupling the sample collecting portion 90 to the sample housing portion 80. The device 10 may be moulded or otherwise suitably manufactured from plastic or other suitable material. Optionally, the device 10 is a single, one-piece unitary construct which may be formed from translucent, transparent or opaque material. Optionally in the presently illustrated embodiment, the device 10 is a plastic moulded, unitary construct which is formed of transparent plastics material. In Figure 2, there is shown a top-view of a second side of the microscope-ready sample collecting device 10 and a cross-section of the device 10 taken along the line A-A.

The device 10 is a generally planar construct having a first primary surface 12a/18a/16a which is shown facing outwards in Figure 1. The device 10 has a second primary surface 12b/18b/16b which is shown facing outwards in Figure 2. The device 10 is moulded and as such is a three-dimensional form, albeit the device 10 has a generally planar body 12a/12b, defined by: a first surface 12a; a second surface 12b; a first end wall 20c; a first side wall 20a; a second side wall 20b; and a partial second end wall 20d. The first and second side walls 20a, 20b are, optionally, a similar length to one another and have a similar depth as each other. The minimum thickness of the device 10 is the distance between the first surface 12a and second surface 12b. The minimum thickness may be between about 0.5mm and about 4mm. Optionally, the depth of the first and second side walls 20a, 20b is between about 5mm and about 20mm. In otherwords, the side walls 20a, 20b and end walls 20c, 20d extend beyone the plane of the second surface 12b of the generall planar body 12a/12b and create a cavity 'C'.

Optionally the first and/or second side wall 20a, 20b is provided with a feature to facilitate gripping, such as but not limited to: one or a series of ribs, bumps, undulations, perturbations, detents and/or indentations. The second end wall 20d is a partial end wall and is interrupted by a hinge element 24 that connects the generally planar body 12a/12b to a second, sample collecting portion 90 of the device 10.

Optionally, the sample collecting portion 90 of the device 10 comprises a neck portion 18a/18b. A first major surface 18a of the neck portion 18a/18b may comprise a feature to facilitate gripping, such as but not limited to: one or a series of ribs, bumps, undulations, perturbations, detents and/or indentations. A second major surface 18b of the neck portion 18a/18b is shown in Figure 2. The neck portion 18a/18b has a depth between the first and second major surfaces 18a, 18b, which may be between about 0.5mm and about 4mm. The neck portion 18a/18b may optionally comprise side walls 63 to add rigidity and strength to the neck portion 18a/18b.

The sample collecting portion 90 further comprises a head portion 16a/16b/36 (also referred to hereing as sample-collecting head 16a/16b). The head portion 16a/16b/36 comprises a first major surface 16a and a second major surface 16b. The first and second major surfaces 16a, 16b face opposite directions and are edged by a wall 36. The wall 36 extends from the neck portion 18a/18b and extends around the first and second major surfaces 16a, 16b. The head portion 16a/16b/36 has a first minimum depth between the first and second major surfaces 16a, 16b, which may be between about 0.5mm and about 4mm; and a second maximum depth which is the depth of the wall 36, which may be between about 5mm and about 20mm.

The head portion 16a/16b comprises a means for collecting a sample, which may also be referred to as a sample-collecting element. Optionally, in the presently illustrated example, the means for collecting a sample is an adhesive applied to the second major surface 16b.

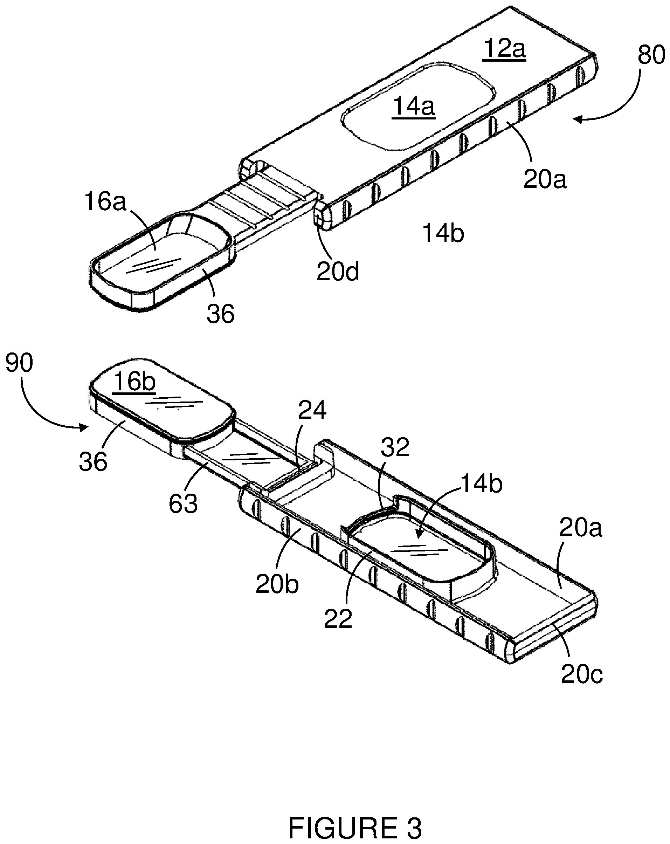

The hinge element 24, foldably couples the sample collector portion 90 of the device 10 to the sample housing portion 80. Optionally, the hinge element 24 may take the form of a moulded, integrally formed, living hinge 24 as shown in Figures 2 and 3. Other suitable hinge mechanisms may be employed in other embodiments; however, beneficially, an intergrally moulded, living hinge 24 faciliates manufacture and allows the device 10 to be moulded as a single-piece, unitary construct, using a single mould, and requires no additional, post-forming assembly or parts.

Formed on the first major side 12a of the generally planar body 12a/12b of the sample housing portion 80, is a sample-viewing window 14a. Formed on the second major side 12b of the generally planar body 12a/12b of the sample housing portion 80 is a sample housing 14b. As viewed from the first side of the device 10, the sample-viewing window 14a is defined on or within the first surface 12a of planar body 12a/12b by shallow moulding. As viewed from the second side of the device 10, the sample housing 14b is formed on the second surface 12b of the planar body 12a/12b and is defined by a frame or wall 22 that is upstanding from the second surface 12b of the device 10. The wall 22 defining the a sample housing 14b is shaped, sized, formed and configured for receiving at least part of the sample-collecting head 16a/16b/36 of the sample collecting portion 90, in such a way that the collected sample can be securely retained and housed therein. The wall 22 defining the a sample housing 14b is optionally a similar size and shape to the shallow moulding defining the sample-viewing window 14a. The wall 22 framing and defining the sample housing 14b has a depth that is similar to a depth or thickness of the wall 36 of the sample collector head 16a/16b/36 of the sample collector portion 90, such that the sample collector head 16b/16b/36 can be received, at least partially, within and retained within the sample housing 14b.

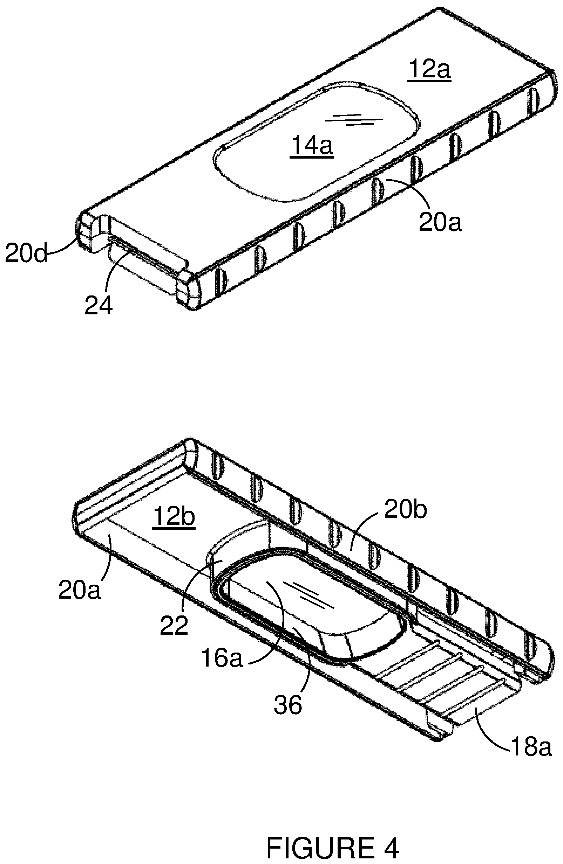

Optionally, the depth of the wall 22 is between about 5mm and about 20mm and does not extend beyond the and second side walls 20a, 20b. In this way, a collected sample disposed on the second surface 16b of the sample collector head 16a/16b/36 can be presented such that it is visible and can be inspected through the sample-viewing window 14a. The folded closed device 10 (see Figure 4) is a generally planar generally rectangular unit that has an overall depth of between about 5mm and about 20mm. Optionally, the wall 22 defining the sample housing 14b and the sample collector head 16b/16b co-operate, interact, or otherwise mechanically fit together in such a way that a collected sample is protected, such that contamination and damage of the collected sample is avoided.

To facilitate collection of a sample, the device 10 may comprise a collection feature associated with the collector head 16a/16b/36. In the present embodiment the second major surface 16b of the collector head 16a/16b/36 has an adhesive coating applied thereon to which the sample can adhere when collected.

Optionally, to faciliate the protection of the collected sample, once held on the second major surface 16b and once housed within the sample housing 14b, the device 10 comprises a connection means 22/32/36 associated with the sample housing 14b and/or the sample-viewing window 14a and/or the the sample collector head 16a/16b/36.

The hinge element 24 allows the sample collector 16a/16b to be foldable relative to the body 12a/12b such that once a sample has been collected using the sample collector head 16a/16b/36, the folded sample collector portion 90 presents the collected sample in the sample housing 14b such that the sample is protected, secure and can be observed through the sample-viewing window 14a defined within the body 12a/12b. In this way, the device 10 of the present invention enables the sample to be disposed in readiness for microscope analysis almost immediately after collection of the sample. Thus avoiding the need for several processing steps in the laboratory involved in the removal of the sample and transfer of the sample for microscope analysis. The device 10 also cuts down on the amount of apparatus required - only a single device is required rather than both a screw-top tube and a microscope slide as in the prior art. The device 10 is also slimline and compact - see Figure 4 - and less bulky than the screw-top tube of the prior art and thus can more economically be sent by post to the laboratory. Compared to the screw-top tube of the prior art the device 10 of the present invention also provides for a greater degree of physical protection of the sample whilst also reducing the opportunity for sample contamination because the sample is more safely and securely contained within the sample housing 14b almost immediately after its collection.

As is shown in Figure 4, once the sample collector portion 90 is folded using the hinge element 24, such that the sample collector head 16a/16b/36 is disposed within the sample housing 14b, the sample-viewing window 14a is in spaced apart facing relationship with the second major surface 16b with the sample carefully contained within the space between the the sample-viewing window 14a and the second major surface 16b. A connection means 22, 32 associated with the sample-viewing housing 14b and/or the sample collector head 16a/16b/36 is operable to secure the sample collector head 16a/16b in the folded, micorscope-ready position. Optionally in the present invention a friction-fit between the wall 36 of the sample collector head 16a/16b/36 and the wall 22/32 defining the sample housing 14b is sufficiently tight that once the sample collector head 16a/16b has been fitted into position (as shown in Figure 4), the sample is secured in a substantially air-tight closed space surrounded by the second major surface 16b, viewing window 14a and walls 36, 22/32. Optionally the connection means 22/36 may snap-fit or audibly "click" into place to indicate to a user secure location of the sample collecting portion 90 within the sample housing 80.

As an option, the wall 22/32 defining the the sample housing 14b comprises a recessed section 32. This is best seen in the bottom image of Figure 3. The recessed section 32 provides a space for the neck 18a/18b of the sample collector portion 90 to be seated.

Once a sample of the test site has been taken, a user of the device 10 should not touch the second major surface 16b as this may cause contamination of or damage to the sample and/or may cause the user to become infected. Preferably, the user will only contact the first surfaces 12a, 16a, the side walls 20a, 20b or end wall 20c as they manipulate the two hinged sections: the sample collector portion 90; and sample housing 80 into the folded, closed condition. The relative movement of the two hinged sections 90, 80 of the device 10 can be done in a number of safe and appropriate ways, to guide the sample collector portion 90 over and into the sample housing 80 such that the sample collector head 16a/16b/36 is seated within the sample housing 14b and such that the neck portion 18a/18b is stowed between the side walls 20a, 20b to form a generally rectangular, planar, microscope slide.

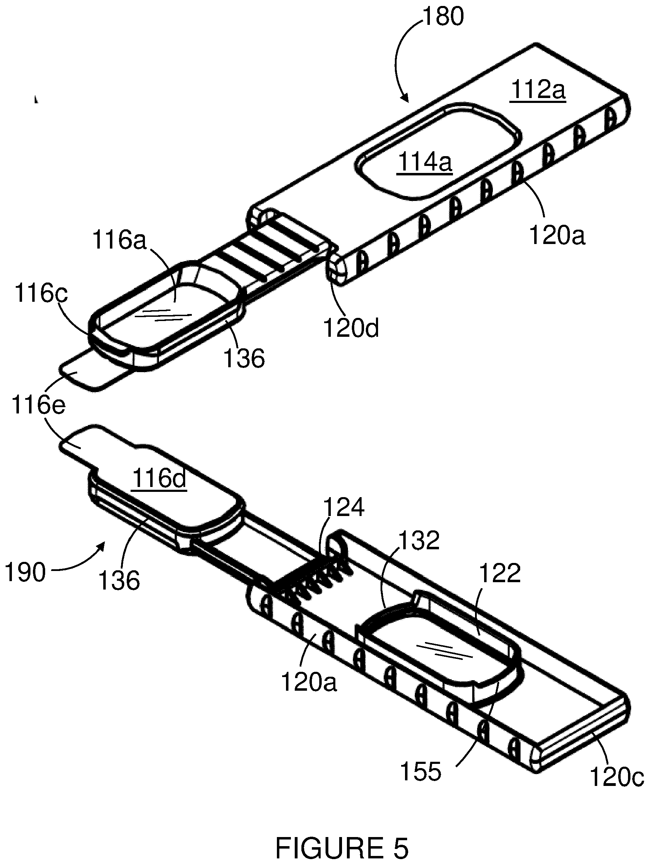

Referring now to Figures 5 and 6; 7; and 8, there are shown additional embodiments of the device 10 of the present invention. In the alternative illustrated embodiments, like numerals have, where possible, been used to denote like parts, albeit with the addition of the prefix "100", "200" and "300" to indicate that these features belong to the additional embodiments respectively. For conciseness, only the differences in the detail or operation of the additional second, third and fourth embodiments are described in detail.

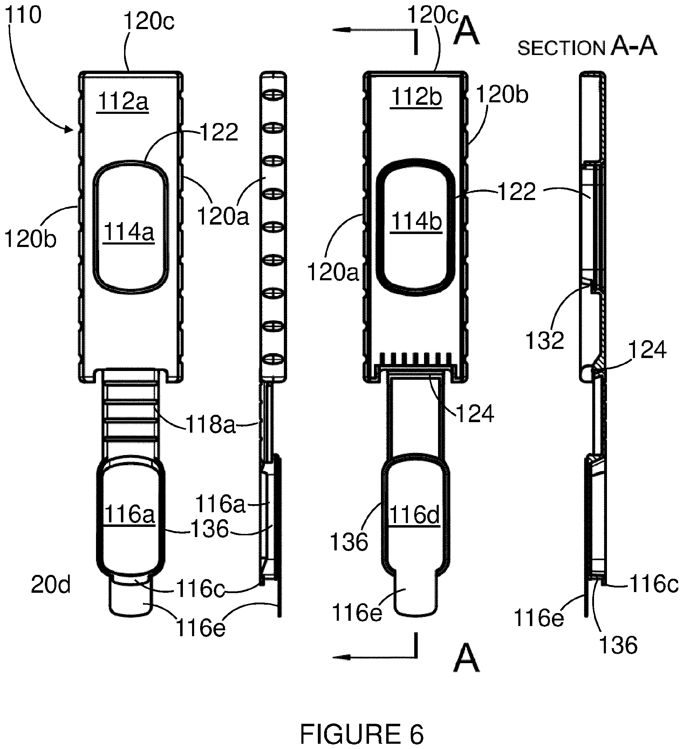

In Figures 5 and 6, the device 110 is provided with a pealable sealing layer 116d/116e disposed over the second major surface 116b of the sample collector head 116a/116b/136. A tab 116c extends from the wall 136 of the sample collector head 116a/116b/136. The wall or frame 122 is provided with a second recess 155 (see Figure 5).

Optionally, the pealable sealing layer 116d/116e is shaped similarly to the shape of the second major surface 116b; albeit, the pealable sealing layer 116d/116e may have an overhanging tab 116e which can be grasped by a user for facilitating removal of the pealable layer 116d/116e. The pealable sealing layer 116d/116e protects an adhesive coating (not illustrated) which is applied to the second major surface 116b. The pealable sealing layer 116d/116e prevents, dust, dirt or other artefacts unrelated to the diagnostic test being carried out, from being adhered to the second major surface 116b. The second major surface 116b is used to test an area of a patient for diagnostic purposes. In the application of pin-worm detection, the area being tested may be a patient's anal area and the pealable sealing layer 116d/116e will be removed only just before that site is tested. Once the pealable sealing layer 116d/116e has been removed - optionally by a user grasping the overhanging tab 116e - the test is conducted by pressing the adhesive-coated second major surface 116b against the patient.

Once a sample of the test site has been taken, a user of the device 110 should not touch the second major surface 116b as this may cause contamination of or damage to the sample and/or may cause the user to become infected. Preferably, the user will only contact the first surfaces 112a, 116a, the side walls 120a, 120b or end wall 120c as they manipulate the two hinged sections: the sample collector portion 190; and sample housing 180 into the folded, closed condition. The relative movement of the two hinged sections 190, 180 of the device 110 can be done in a number of safe and appropriate ways. Optionally, the tab 116c can be pressed by a user's thumb to guide the sample collector portion 190 over and into the sample housing 180 such that the sample collector head 116a/116b/136 is seated within the sample housing 114b and such that the neck portion 118a/118b is stowed between the side walls 120a, 120b to form a generally rectangular, planar, microscope slide.

The tab 116c additionally, optionally forms part of the connection means 122/132/136/116c and may snap-fit or "click" into place between edges defined by the second recess 155. Additionally or alternatively, the tab 116c may be provided to allow for re-opening of the device 110 should it be required. The tab 116c can be engaged to flip the sample collector head 116a/116b/136 out of the housing 114b.

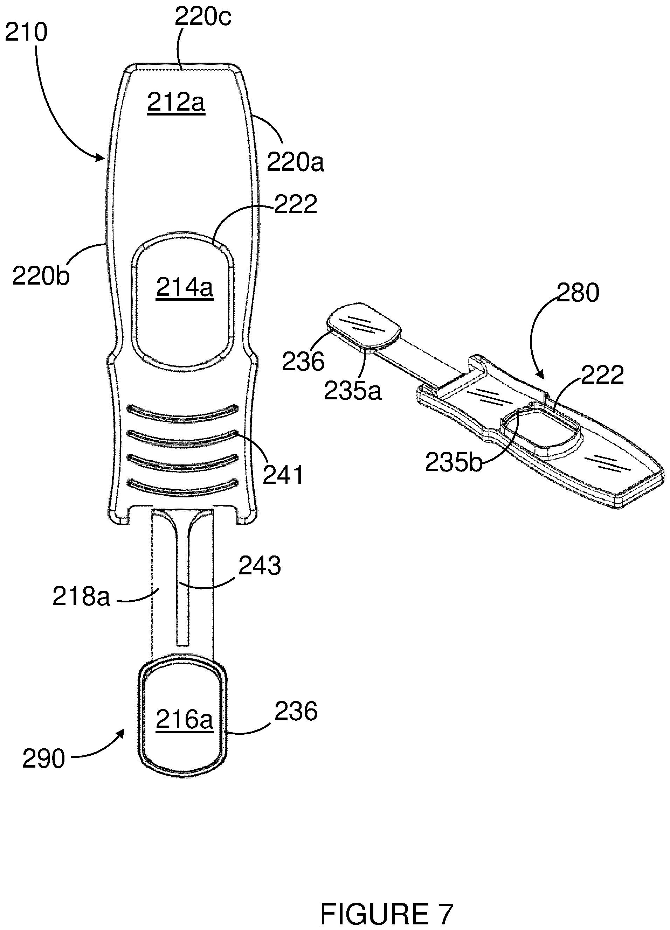

In Figure 7 a device 210 is shown in which the side walls 220a, 200b are shaped in a more ergonomic manner to assist with handling of the device 210, particularly during the collection of a sample. The sampling head 216a/216b/236 in this arrangement does not necessarily comprise an adhesive layer and sampling may be achieved simply by swabbing a test site, by contacting the second major surface 216b of the sampling head 216a/216b/236 against a sample to be tested. Optionally the second major surface 216b of the sampling head 216a/216b/236 may be dragged over a test site multiple times to ensure that a sufficient quantity of sample is collected. Then the sample is stowed securely within the sample housing 214b in a similar way as that described above.

Additionally or aternatively, in the embdiment of Figure 7, in the device 210 shown, the neck portion 218a may be strengthend by a rib 243. Additoinally or alternatively, ribs or other surface details 241 may be provided on the first major surface 212a of the body 212a/212b in order to facilitate gripping during the taking of the sample from a patient. This feature is synergistic with the use of the sampling head 216a/216b/236 for collecting a sample of a pathogen where a test site may be swabbed multiple times and it is important for the user to carefully control the use of the device 210 and to ensure that the sampling head 216a/216b/236 does not come into contact with any other surfaces which could cause contamination of the sample and/or infection of another.

The sampling head 216a/216b/236 again snap-fits or "clicks" into place with the wall 236 fitting tightly and sealingly within and against the wall 222 of the housing 214b. Optionally, in this arrangement, the device 210 is provided for the sampling of a pathogen or fluid and the sampling head 216a/216b/236 can be sealed within the sample housing 214b. Optionally, the tightness of the seal may be substantially or completely air tight. In some embodiments, for various applications the seal between the wall 236 of the sampling head 216a/216b/236 and the wall 222 of the the sample housing 214b may be sufficiently tight that the sample is hemetically sealed within the sample housing 214b. To assist with the tight seal, sealing elements 235a and 235b may be provided. The sealing elements 235a, 235b may comprise a first sealing element 235a disposed circumferentially about the sampling head 216a/216b/236 wall 236. The first sealing element 235a may be disposed about the sampling head 216a/216b/236 wall 236 in an uninterrupted and contiguous manner. The first sealing element 235a may be a moulded feature formed of the same material as the rest of the device 210 and formed during the moulding process as the rest of the device 210. Alternatively, the first sealing element 235a may be formed from a different material, for example, rubber or neoprene and may be seated within a ridge or groove or seated against a flange moulded into the outside of wall 236. The second sealing element 235b may be disposed within the sampling housing 214b wall 222 in an uninterrupted and contiguous manner. The second sealing element 235b may be a moulded feature formed of the same material as the rest of the device 210 and formed during the moulding process of the rest of the device 210. Alternatively, the second sealing element 235b may be formed from a different material, for example, rubber or neoprene and may be seated within a ridge or groove or seated against a flange moulded into the inside surface of the wall 222.

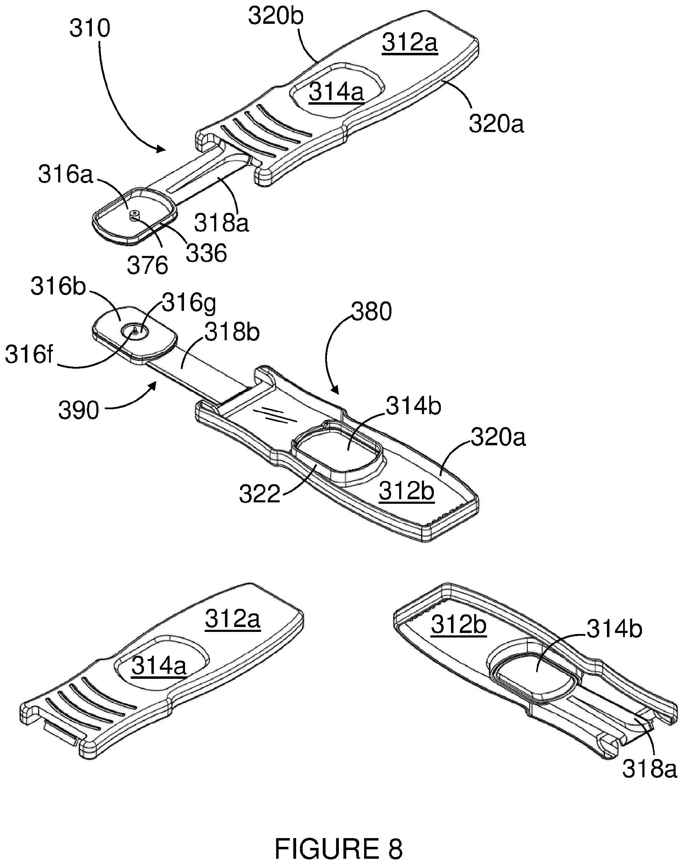

In Figure 8, a device 310 is shown which is configured for the taking of a (small) blood sample. The device 310 shown has a different form of collection feature 316f which takes the form of a sharp or pointed or pricking member 316f which can prick skin or tissue to release blood which can pool in the small well 316g surrounding the sharp or pointed member 316f. The well 316g may be a moulded feature; the sharp or pointed head 316f may be formed of a separate material and may or may not be intergrally moulded.

It can be appreciated that various changes may be made within the scope of the present invention, for example, in other embodiments of the invention it is envisaged that various features shown in one embodiment could be used conjunction shown in another embodiment which combination of features is not specifically illustrated, provided such features are compatible.

## Claims

1. A microscope-ready sample collecting device (10, 110, 210, 310) comprising a sample housing portion (80, 180, 280, 380); a sample collecting portion (90, 190, 290, 390) comprising a sample collecting head (16a/16b/36, 116a/116b/136, 216a/216b/236, 316a/316b/336); and a hinge element (24, 124, 224, 324) between the sample housing portion (80, 180, 280, 380) and the sample collecting portion (90, 190, 290, 390) for foldably coupling the sample collecting portion (90, 190, 290, 390) to the sample housing portion (80, 180, 280, 380), the sample housing portion (80, 180, 280, 380) comprising:
**i)** a generally planar main body (12a/12b; 112a/112b; 212a/212b; 312a/312b) and first and second opposing side walls (20a, 20b; 120a, 120b; 220a, 220b; 320a, 320b);
**ii)** a sample-viewing window (14a; 114a; 214a; 314a) defined on or in a first surface (12a; 112a; 212a; 312a) of the generally planar main body (12a/12b; 112a/112b; 212a/212b; 312a/312b); and
**iii)** a sample-housing (14b; 114b; 214b; 314b) defined by a wall (22; 122; 222; 322) upstanding from a second surface (12b; 112b; 212b; 312b) of the generally planar main body (12a/12b; 112a/112b; 212a/212b; 312a/312b);
wherein the hinge element (24, 124, 224, 324) allows the sample collecting head (16a/16b/36, 116a/116b/136, 216a/216b/236, 316a/316b/336) to be folded relative to the sample housing portion (80, 180, 280, 380) such that the sample collecting head (16a/16b/36, 116a/116b/136, 216a/216b/236, 316a/316b/336) can be received within the sample housing (14b; 114b; 214b; 314b) such that a sample collected using the sample collecting head (16a/16b/36, 116a/116b/136, 216a/216b/236, 316a/316b/336) can thereby be presented relative to the sample-viewing window (14a; 114a; 214a; 314a) such that almost immediately after collection, a sample is disposed in readiness for microscope analysis;
wherein said wall (22; 122; 222; 322) and/or said sample housing (14b; 114b; 214b; 314b) are shaped and configured to enclose the sample collecting head (16a/16b/36, 116a/116b/136, 216a/216b/236, 316a/316b/336); and said sample collecting head (16a/16b/36, 116a/116b/136, 216a/216b/236, 316a/316b/336) and said sample-housing (14b; 114b; 214b; 314b) are configured to co-operably mechanically fit together for protecting a collected sample and for mitigating against contamination and damage of the collected sample.

2. A device (10, 110, 210, 310) according to claim 1, wherein said sample collecting head (16a/16b/36, 116a/116b/136, 216a/216b/236, 316a/316b/336) and said sample-housing (14b; 114b; 214b; 314b) are configured to co-operably mechanically fit together by a connection means (22/32/36; 122/132/136; 222/232/236; 322/336) associated with the sample housing (14b; 114b; 214b; 314b) and/or the sample collecting head (16a/16b/36, 116a/116b/136, 216a/216b/236, 316a/316b/336); the connection means (22/32/36; 122/132/136; 222/232/236; 322/336) associated with the sample housing (14b; 114b; 214b; 314b) and/or the sample collecting head (16a/16b/36, 116a/116b/136, 216a/216b/236, 316a/316b/336) being operable once the device (10, 110, 210, 310) is folded and enables the sample collecting head (16a/16b/36, 116a/116b/136, 216a/216b/236, 316a/316b/336) to be connected to the wall (22; 122; 222; 322) of the sample housing (14b; 114b; 214b; 314b) for securing the sample collecting head (16a/16b/36, 116a/116b/136, 216a/216b/236, 316a/316b/336) in the folded, micorscope-ready position.

3. A device (10, 110, 210, 310) according to claim 2 wherein the connection means (22/32/36; 122/132/136; 222/232/236; 322/336) takes the form of co-operating elements that snap-fit between the sample collecting head (16a/16b/36, 116a/116b/136, 216a/216b/236, 316a/316b/336) and the upstanding wall (22; 122; 222; 322) of the sample-housing (14b; 114b; 214b; 314b) portion.

4. A device (10, 110, 210, 310) according to any claim 1, 2 or 3, wherein the sample collecting head (16a/16b/36, 116a/116b/136, 216a/216b/236, 316a/316b/336) is stadium-shaped or paddle-shaped.

5. A device (10, 110, 210, 310) according to claim 4 wherein the sample-viewing window (14a; 114a; 214a; 314a) is shaped and sized correspondingly to the shape and size of the stadium-shaped or paddle-shaped sample collecting head (16a/16b/36, 116a/116b/136, 216a/216b/236, 316a/316b/336).

6. A device (10, 110, 210, 310) according to any preceding claim wherein the sample collecting portion (90, 190, 290, 390) comprises a neck (18a/18b; 118a/118b; 218a/218b; 318a/318b) and wherein the neck (18a/18b; 118a/118b; 218a/218b; 318a/318b) has a narrower width than a maximum width of the sample collecting head (16a/16b/36, 116a/116b/136, 216a/216b/236, 316a/316b/336).

7. A device (10, 110, 210, 310) according to claim 6, wherein the hinge element (24, 124, 224, 324) is provided by a living hinge moulded between the neck (18a/18b; 118a/118b; 218a/218b; 318a/318b) and the planar main body (12a/12b; 112a/112b; 212a/212b; 312a/312b).

8. A device (10, 110, 210, 310) according to claim 6 or 7, wherein on an outer surface, the neck (18a/18b; 118a/118b; 218a/218b; 318a/318b) comprises one or more ribs, ridges or other grip-enhancing mouldings (243).

9. A device (10, 110, 210, 310) according to any preceding claim, wherein the sample collecting head (16a/16b/36, 116a/116b/136, 216a/216b/236, 316a/316b/336) comprises an adhesive coating on at least one side; or wherein the sample collector comprises a sharp portion (316f).

10. A device (10, 110, 210, 310) according to any preceding claim, wherein the first and second opposing side walls (20a, 20b; 120a, 120b; 220a, 220b; 320a, 320b) of the generally planar main body (12a/12b; 112a/112b; 212a/212b; 312a/312b) each have a height of between about 5mm and about 20mm.

11. A device (10, 110, 210, 310) according to any preceding claim wherein on outer surfaces the first and second opposing side walls (20a, 20b; 120a, 120b; 220a, 220b; 320a, 320b) of the generally planar main body (12a/12b; 112a/112b; 212a/212b; 312a/312b) one or more ribs, ridges or other grip-enhancing mouldings are provided.

12. A device (10, 110, 210, 310) according to any preceding claim, whereupon the sample collecting portion (90, 190, 290, 390) being folded into a secure location within the folded sample housing portion (80, 180, 280, 380), and the sample collecting head (16a/16b/36, 116a/116b/136, 216a/216b/236, 316a/316b/336) being properly disposed for inspection through the sample-viewing window (14a; 114a; 214a; 314a), the sample collecting head (16a/16b/36, 116a/116b/136, 216a/216b/236, 316a/316b/336) is secured to the sample housing (14b; 114b; 214b; 314b) for maintaining the collected sample in the correct location for analysis by microscope.

13. A device (10, 110, 210, 310) according to any preceding claim, wherein the sample housing (14b; 114b; 214b; 314b) has a depth defined by the height of the upstanding wall (22; 122; 222; 322); and wherein the upstanding wall (22; 122; 222; 322) has a depth of between about 5mm and about 25mm.

14. A device (10, 110, 210, 310) according to any preceding claim when dependent upon claim 2, wherein upon using the connection means (22/32/36; 222/232/236; 322/336) to secure the folded sample collecting head (16a/16b/36, 116a/116b/136, 216a/216b/236, 316a/316b/336) in the sample housing (14b; 114b; 214b; 314b), the connection means (22/32/36; 222/232/236; 322/336) cannot readily, by hand, be disconnected, thereby providing a tamper-evident feature to validate and confirm the integrity of the protected, collected sample.

15. A device (10, 110, 210, 310) according to any preceding claim 2 to 13 when dependent upon claim 2, wherein a release tab (116c) is provided, such that upon using the connection means (122/132/136) to locate the folded sample collecting head (16a/16b/36, 116a/116b/136, 216a/216b/236, 316a/316b/336) in the sample housing (14b; 114b; 214b; 314b), the release tab (116c) is operable to disengage the connection means (122/132/136).
